# EUROPEAN PATENT SPECIFICATION

(11) **EP 3 422 956 B1**
(45) Date of publication and mention of the grant of the patent: **02.12.2020**
(21) Application number: 17711485.7
(22) Date of filing: 03.03.2017
(51) Int. Cl.: A61B 17/04, A61B 17/064, A61B 17/86

(54) **VARIABLE LENGTH ANCHOR**
ANKER MIT VARIABLER LÄNGE
ANCRAGE DE LONGUEUR VARIABLE

(30) Priority: 03.03.2016 US 201662303012 P
(43) Date of publication of application: 09.01.2019
(73) Proprietor: Smith & Nephew, Inc, Memphis, TN 38116 (US)
(72) Inventor: ROGERS, Jon-Paul, North Smithfield Rhode Island 02896 (US); O'CONNOR, Paul, Norfolk Massachusetts 02056 (US); CALLAHAN, Timothy, Wrentham Massachusetts 02093 (US)
(74) Representative: Smith & Nephew
(86) International application number: PCT/US2017/020574
(87) International publication number: WO 2017/152000

(56) References cited:
- EP-A1- 2 505 142
- WO-A1-01/10312
- US-A1- 2004 098 053
- US-A1- 2005 143 734
- US-A1- 2013 267 998

## Description

### FIELD OF THE INVENTION

The present invention relates generally to the field of anchors, and more particularly relates to improved anchors of inserting anchors that may be fractured or snapped apart after insertion to reduce the length of the anchors. Some embodiments may include a distal portion of an inserter that provides a removable support element to an anchor, for example during insertion and manipulation.

### BACKGROUND

It is conventional to use tissue anchors to secure sutures, soft tissue grafts, or other components in tissue such as bone. For example, a blind hole or other tunnel in bone may be created in which one or more of a suture, soft tissue graft, or other component is to be coupled. A common, conventional technique is to insert an "interference screw" into a blind hole or bone tunnel with a suture, soft tissue graft, or other component. It is often difficult to insert the interference screw or other, conventional implant into the bone tunnel with the suture, soft tissue graft, or other component without lacerating, crushing, twisting, or otherwise harming the suture, soft tissue graft, or other component. Such a technique is particularly difficult where the anchor is a threaded anchor, which is often the case with prior art devices. The rigid and specific, inflexible characteristics and sizes of many prior art anchor devices lead to a requirement for complex calculations related to bone quality, bone hardness, graft size, hole size, screw size, screw material, and thread design to ensure effective graft attachment.

It would be advantageous to provide an anchoring system capable of securing a suture, soft tissue graft, or other component in a blind hole or other bone tunnel without imposing potentially damaging friction or stress on the suture, soft tissue graft, or other component. Devices of improved flexibility and adjustable size may provide for simplified calculations related to bone quality, bone hardness, graft size, hole size, screw size, screw material, and thread design. It may additionally be advantageous to provide an anchoring system that allows for adjustment of the suture, soft tissue graft, or other component after the anchor has been substantially fully inserted. Further, it may be advantageous to provide an anchor that may be readily reduced in length after being substantially fully inserted in a blind hole, especially where a number of different lengths for the implant may be chosen by a user of the anchoring system. Adjustable length anchors require fewer part numbers, or Stock Keeping Units, while providing the same clinical efficacy as systems with multiple length anchors. It may also be advantageous to provide anchors that take up a reduced amount of the cross-sectional area of a hole in which an anchor is to be placed so that a larger soft tissue graft and larger area for ingrowth and healing may be provided.

US 2005/143734 A1, US 2004/098053 A1, US 2013/267998 A1, WO01/10312 A1, and EP 2505142 A1 each disclose an anchor with a body that is segmented at one or more locations along its length.

### SUMMARY

An embodiment of the invention is an anchoring system that includes an anchor and an inserter. The anchor includes an anchor body with a distal end, a proximal end, and a length between the distal end and the proximal end, and one or more anti-backout features along the length of the anchor body configured to resist proximal movement of the anchor when the anchor has been inserted in a hole with a diameter equal to or less than a maximum diameter of the anchor. The anchor body is segmented at one or more locations along its length such that the length of the anchor may be reduced by removing the proximal end and a portion of the length of the anchor body. The inserter of the anchoring system includes a handle and a shaft coupled to the handle wherein the shaft includes a connecting mechanism configured to releasably couple with the anchor. The connecting mechanism includes a distal end of the shaft that extends along and contacts a portion of the anchor to support the anchor. The anchor body of the anchor has a cannulation. The distal end of the shaft is sized to fit within the cannulation in the anchor to provide support to the anchor. The anchor body is segmented by one or more couplings between snap together components, wherein the snap together components are configured to be detached at the one or more couplings to reduce the length of the anchor body.

Another example of the disclosure is a frangible anchor with an anchor body with a distal end, a proximal end, and a length between the distal end and the proximal end. The anchor body may be segmented at one or more locations along its length such that the length of the frangible anchor may be reduced by removing the proximal end and a portion of the length of the anchor body.An additional example of the disclosure is an anchor having an anchor body with a distal end, a proximal end, and a length between the distal end and the proximal end. The anchor body may include a cross-sectional shape that has a larger diameter in first direction and a relatively smaller diameter in a second direction that is transverse to the first direction such that when the anchor is inserted in a hole substantially the same diameter as the larger diameter a first space exists in a direction of a first side of the smaller diameter through which a suture may be passed and a second space exists in a direction of a second side of the smaller diameter through which a soft tissue graft coupled to the suture may be passed. The anchor body may also be segmented at one or more locations along its length such that the length of the anchor may be reduced by removing the proximal end and a portion of the length of the anchor body.

Still another example of the disclosure is a method, which is not part of the invention, of inserting a soft tissue graft into a bone. The method example may include providing a suture for coupling to the soft tissue graft and passing the suture around a portion of an anchor wherein the anchor includes a body with a cross-sectional shape that has a larger diameter in first direction and a relatively smaller diameter in a second direction that is transverse to the first direction such that when the anchor is inserted in a hole in the bone substantially the same diameter as the larger diameter a first space exists in a direction of a first side of the smaller diameter through which a suture may be passed and a second space exists in a direction of a second side of the smaller diameter through which a soft tissue graft coupled to the suture may be passed. Method examples may also include inserting the anchor into the bone such that the larger diameter of the anchor firmly engages with the bone and until the soft tissue graft being advanced with the anchor is tensioned to a desirable degree.

Yet another example of the disclosure is a method of inserting an anchor into a bone. The method example may include coupling the anchor and an inserter by placing a distal end connecting mechanism of the inserter into a cooperating portion in the anchor to provide support to the anchor, inserting the anchor into the bone, decoupling the inserter and the anchor, and breaking off a proximal end and a portion of a length of the anchor.

### BRIEF DESCRIPTION OF THE DRAWINGS

FIG. 1 is a perspective view of an embodiment of an anchoring system.
FIG. 2 is a perspective view of an anchor of the anchoring system of FIG. 1.
FIG. 3 is a cross-sectional elevation view of the anchor of FIG. 2.
FIG. 4 is a back elevation view of the anchor of FIG. 2.
FIG. 5 is a side elevation view of the anchor of FIG. 2.
FIG. 6 is a front elevation view of the anchor of FIG. 2.
FIG. 7 is a perspective view of a central portion of an alternate embodiment of an anchor of an anchoring system.
FIG. 8 is a perspective view of an anchor and a distal portion of an inserter of the anchoring system of FIG. 1.
FIG. 9 is a cross-sectional perspective view of the anchor and distal portion of the inserter of FIG. 8.
FIG. 10 is a perspective view of an inserter of the anchoring system of FIG. 1.
FIG. 11 is a perspective view of the anchoring system of FIG. 1 with a soft tissue graft with interwoven anchor suture and a stay suture shown.
FIG. 12 is a side elevation view of the anchor and soft tissue graft with interwoven anchor suture of FIG. 11 being inserted into bone, where the bone is shown cut away in cross-section.
FIG. 13 is a perspective view of the anchor, the soft tissue graft with interwoven suture, and the stay suture of FIG. 11 inserted in bone, and an anchor length reduction tool to be applied to the anchor.
FIG. 14 is a perspective view of the anchor and the soft tissue graft with interwoven anchor suture of FIG. 13 after the anchor has been broken off
FIG. 15 is a side elevation view of the anchor and soft tissue graft with interwoven suture of FIG. 12 after the anchor has been broken off.
FIG. 16 is a perspective view of an anchor and a distal portion of an inserter of an embodiment of an anchoring system.
FIG. 17 is a cross-sectional perspective view of the anchor and distal portion of the inserter of FIG. 16.
FIG. 18 is a side elevation view of the anchor of FIG. 16 and a soft tissue graft with interwoven anchor suture after being inserted into bone, where the bone is shown cut away in cross-section.
FIG. 19 is a perspective view of an anchor and a distal portion of an inserter of an embodiment of an anchoring system.
FIG. 20 is a perspective view of the anchor of FIG. 19.
FIG. 21 is a perspective view of the distal portion of the inserter of FIG. 19.
FIG. 22 is a side elevation view of the anchor of FIG. 19 and a soft tissue graft with interwoven anchor suture after being inserted into bone, where the bone is shown cut away in cross-section.

### DETAILED DESCRIPTION

The provisional application to which the present application claims priority was filed on the same day with U.S. Provisional Application No. 62/303,019, filed March 3,2016, entitled "PUSH-IN ANCHOR AND METHOD".

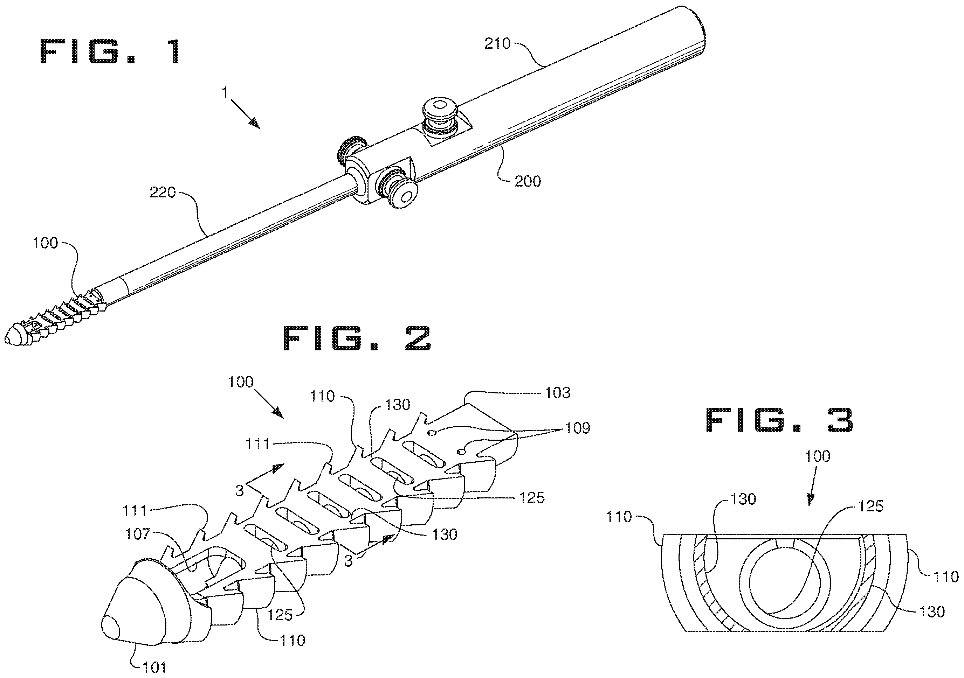

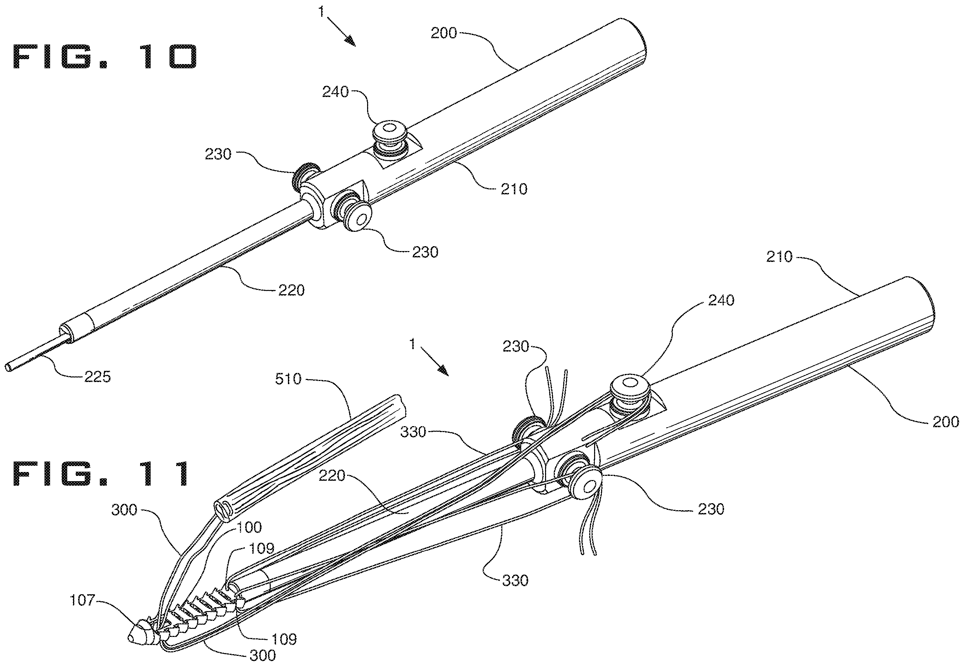

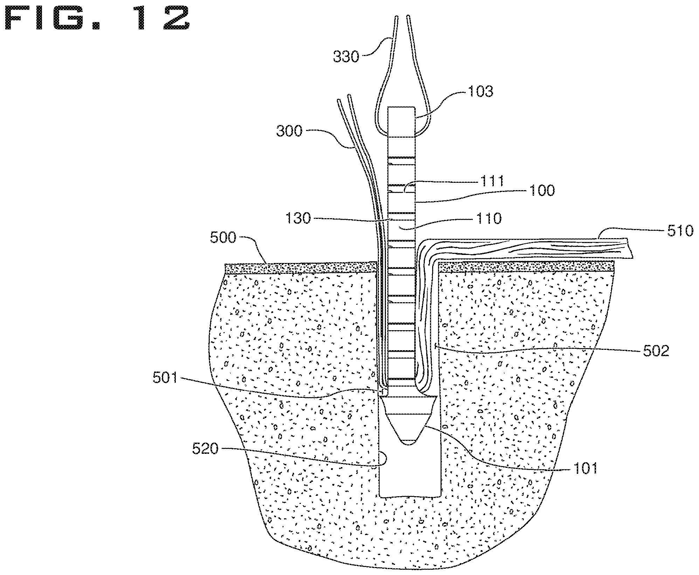

The present invention is disclosed in the appended set of claims. An embodiment of an anchoring system 1 and its component parts and methods of implementation are illustrated in FIGS. 1-15. As shown in FIGS. 1 and 11, the anchoring system 1 may include an anchor 100 and an inserter 200. Various features of the anchor 100 are illustrated in combination in FIGS. 1-6, 8, 9, and 11-15. The anchor 100 shown includes an anchor body with a distal end 101, a proximal end 103, and a length between the distal end 101 and the proximal end 103. The illustrated embodiment also shows anti-backout features in the form of teeth 110 along the length of the anchor body. The teeth 110 shown are configured to resist proximal movement of the anchor 100 when the anchor 100 has been inserted in a hole 520 (FIGS. 12-15) in a bone 500 with a diameter equal to or less than a maximum diameter of the anchor 100. As used in association with the terms maximum diameter and minimum diameter herein, the term "diameter" is a straight line distance through an anchor from one side of the anchor to the other side of the anchor that passes through a cross-sectional central portion of the anchor. A "diameter" as used herein is not limited to an object with a circular or even rounded cross-section.

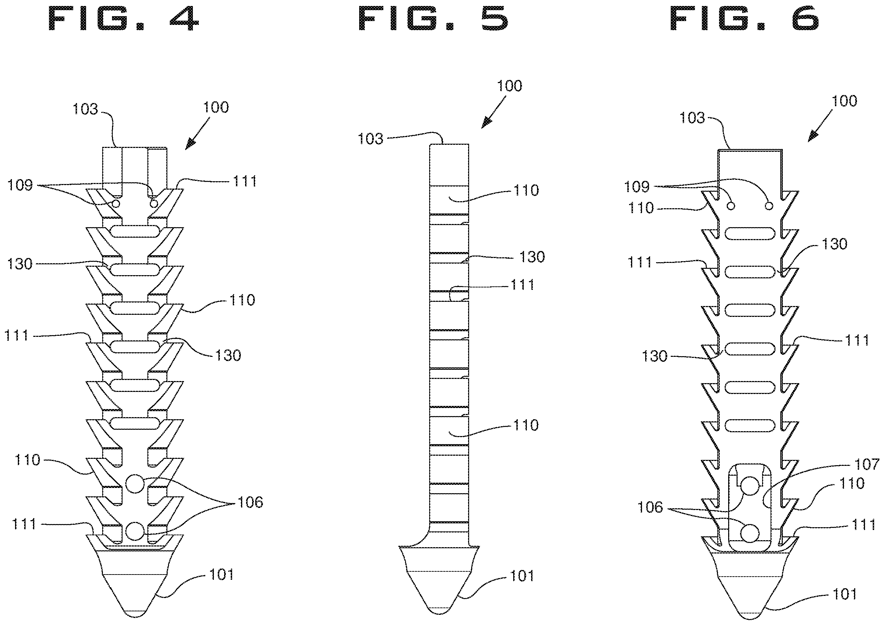

Embodiments of the invention may include an anchor body with a cross-sectional shape that has a larger diameter in a first direction and a relatively smaller diameter in a second direction. For example and without limitation, the anchor 100 has a larger diameter illustrated laterally across FIGS. 4 and 6 and a smaller diameter illustrated laterally across FIGS. 5, 12, and 15. The smaller diameter may be transverse to the first direction such that when an anchor, such as the anchor 100, is inserted in a hole that is substantially the same diameter as the larger diameter, a first space exists in the direction of a first side of the smaller diameter through which a suture may be passed and a second space exists in a direction of a second side of the smaller diameter through which a soft tissue graft coupled to the suture may be passed. For example, as depicted in FIG. 12, a first space 501 exists in the direction of a back side of the anchor 100 through which the anchor suture 300 may be passed. A second space 502 exists in the direction of a front side of the anchor 100 through which the soft tissue graft 510 may be passed. With the anchor 100 in the position illustrated in FIG. 12, the larger diameter of the anchor 100 is engaged with a wall of the hole 520 to secure the anchor 100 relative to the bone 500. The second, transverse direction of the illustrated embodiment is substantially perpendicular to the first direction. However, in other embodiments the directions may not be substantially perpendicular but are transverse to some degree. The distal end 101 of the anchor 100 includes a portion that is substantially round with a diameter in all directions larger than the smaller diameter of the anchor 100. Other embodiments may or may not include a substantially round distal end portion. As used herein, a hole substantially the same diameter as the larger diameter is not considered to be an exact specification, particularly in light of the fact that holes in bone may be relatively imprecise or irregular.

The teeth 110 depicted may be resilient or flexible such that the teeth 110 flex away from the wall of the hole 520 (FIGS. 12-15) when the anchor 100 is inserted into the hole 520. A resilient or flexible tooth may also include an angular edge, such as the angular edge 111, that catches against the wall of the hole 520 if a proximal force is applied to the anchor 100. Anti-backout features of these or other embodiments may include barbs, hooks, spikes, or any other effective mechanism to resist backout of an anchor to which the features are attached. For example and without limitation, anti-backout elements may be triangular, square, pyramid shaped, shark's tooth shaped, and raked to any effective degree.

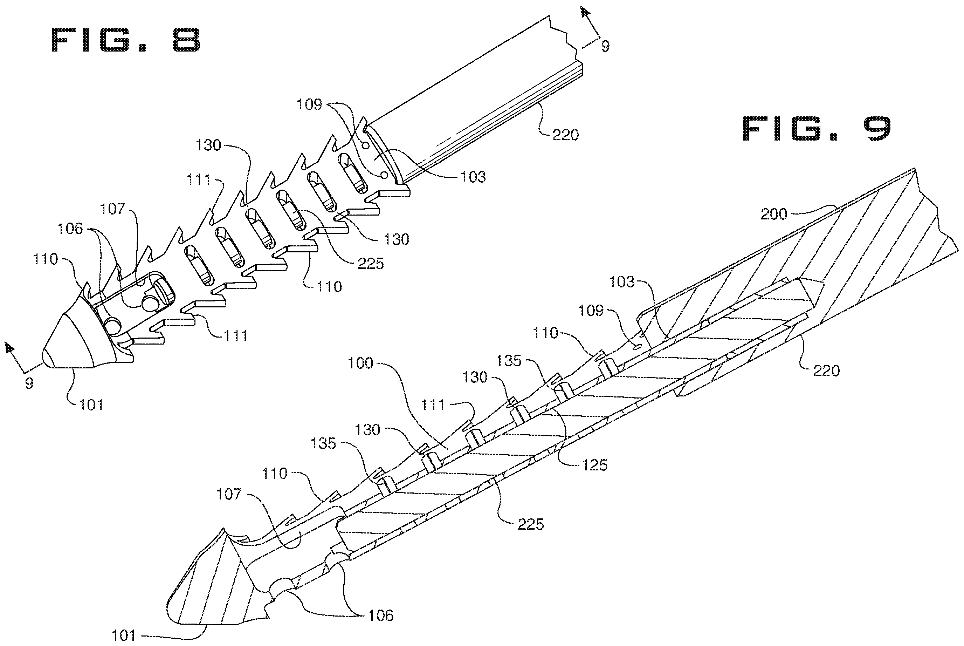

The anchor body of the anchor 100 shown is segmented at multiple locations along its length such that the length of the anchor 100 may be reduced by removing the proximal end 103 and a portion of the length of the anchor body. Segmentations at multiple locations along the anchor body of the anchor 100 are illustrated at least in FIGS. 2-6, 8, 9, and 12-15. The segmentations shown include zones of reduced material thickness 130 that are configured to be fractured to reduce the length of the anchor body of the anchor 100. Zones configured to be fractured may be cross-sectional portions of an anchor that have less material volume compared with other cross-sections of the anchor or may have features specifically designed to fracture more readily. As particularly well illustrated in FIG. 9, the zones of reduced material thickness 130 may include scored lines 135 that further reduce material thickness at specific locations where fractures may be induced. The scored lines 135 may also assist with breaking the anchor 100 more cleanly along a specific path. These or similar scorings, etchings, molded indents or the like, may be employed with various embodiments. The portion of the anchor 100, or other anchor embodiments, to be removed may be of any useful length, and fracture locations generally but not necessarily align with zones of reduced material thickness. For example and without limitation, a fracture location of some embodiments could be dictated by the placement and configuration of a cooperating anchor length reduction tool. The zones of reduced material thickness 130 of the illustrated embodiment may also provide through-growth holes for tissue to penetrate and grow through the anchor 100 and thereby enhance fixation of the anchor 100 over time. Through-growth holes may be included in other embodiments that are not part of zones of reduced material thickness.

The anchor body of the anchor 100 may include numbers to provide an indication of a depth of penetration of the anchor into the hole 520, as described in association with the anchor 2100 herein. Also similar to the embodiment of anchor 2100, depth of penetration may be determined based on numbers of teeth extending above the surface of a bone during a given state of insertion.

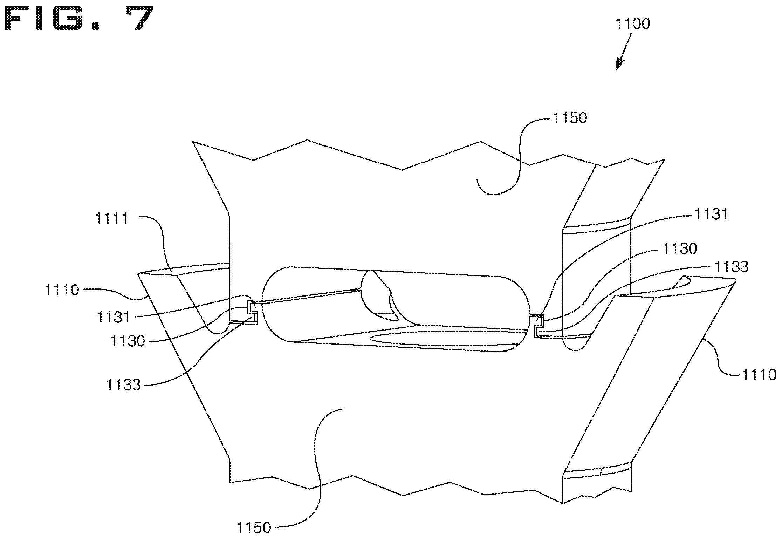

An anchor 1100 is depicted in FIG. 7 that is an embodiment of an anchor of an anchoring system. In this embodiment, the anchor body is segmented by couplings 1130 between snap together components 1150. The snap together components 1150 are configured to be detached at the couplings 1130 to reduce the length of the anchor body 1100. Each of the couplings 1130 includes a base 1131 and a hook 1133 that are configured to be snapped together to form the anchor 1100. The couplings 1130 may include adhesives, some frangible material segment, additional interdigitating components, or may merely be fixed together relatively firmly and held by friction. The anchor 1100 may interact with the inserter 200, sutures 300, 330, and the tissues of the patient similar to the way the anchor 100 is described interacting with such structures herein. Similarly, portions of the anchor 1100 may be detached from one another as described with regard to the anchor 100 herein.

Some features of the inserter 200 are shown in combination in FIGS. 1 and 8-11. The inserter 200 is illustrated in FIG. 10 with a handle 210 and a shaft 220 coupled to the handle 210 wherein the shaft 220 includes a connecting mechanism configured to releasably couple with the anchor 100. In particular, the connecting mechanism illustrated includes a distal end 225 of the shaft 220 that extends along and contacts a portion of the anchor 100 to support the anchor 100 (FIGS. 8 and 9). In some embodiments, such support provides a temporary substantially rigid construct for insertion. The portion of the anchor 100 contacted is a cannulation 125 (FIGS. 2, 3, 9, 13, and 14) in the illustrated embodiment. However, in other embodiments other portions of an anchor may be supported by portions of an inserter to facilitate insertion of an anchor such as the segmented anchor 100 that may benefit from additional support. As shown in FIGS. 8 and 9, the proximal end 103 of the anchor 100 also fits within a portion of the shaft 220 of the inserter 200 to form a part of the connecting mechanism of the inserter 200 in cooperation with the anchor 100. In the particular case of the illustrated embodiment, the anchor 100 benefits from additional support of the distal end 225 of the shaft 220 while insertion forces are being applied to the inserter and anchor construct. After the anchor 100 is inserted, the inserter 200, and particularly the distal end 225 of the inserter 200, may be removed from the anchor 100, as depicted in FIG. 12.

The inserter 200 illustrated in FIGS. 10 and 11 also includes stay suture holders 230 and an anchor suture holder 240. The stay suture holders 230 and the anchor suture holder 240 are shown as conventional wraparound suture holders. However, in other embodiments any other effective mechanism may be used to secure sutures relative to an inserter embodiment of the invention. Stay sutures 330 are shown in FIG. 11 removably coupling between the stay suture holders 230 and the anchor 100. The anchor 100 includes stay suture holes 109 (FIGS. 2, 4, 6, 8, 9, and 13) through which one or more stay sutures 330 may be passed to enable removable coupling between the anchor 100 and inserter 200. Any other effective connecting mechanism may be used to removably couple between an inserter and an anchor of this or other embodiments. For example and without limitation, an anchor of some embodiments may be held to an inserter by friction or by some mechanical feature such as a snap, hook, or ball and detent, rather than or in addition to a stay suture.

A soft tissue graft 510 is illustrated in FIGS. 11-15, 18, and 22. The soft tissue graft of this or other embodiments may be a tendon, ligament, muscle, cartilage, or other tissue or synthetic material to be secured. In FIG. 11, an anchor suture 300 has been whipstitched to the soft tissue graft 510. The anchor suture 300 illustrated includes two strands of suture 300 that have been threaded through suture holes 106 (FIGS. 4, 6, 8, and 9) and connected with the anchor suture holder 240. The anchor 100 also includes a pocket 107 (FIGS. 2, 6, 8, 9, and 11) adjacent to the suture holes 106. The pocket is configured to receive an end portion of the soft tissue graft 510 when distal ends of the anchor suture 300 are pulled distally relative to the inserter 200 and the anchor 100. The pocket 107 may be useful to more effectively capture an end of the soft tissue graft 510 and secure the soft tissue graft 510 relative to the anchor 100. Other embodiments may include other structures for enhancing capture of a soft tissue graft or may not include any additional structures for capturing a soft tissue graft relative to an anchor. In other embodiments, graft tissue, a patient's tissue, or sutures of any type, or a combination of such elements may be passed through an opening such as one or both of the suture holes 106 for anchoring. Sutures of the anchoring system may be any type of suture, for example and without limitation, a monofilament, multistrand, or woven construct. A suture may be passed through an anchor while the anchor is outside of a patient's body, or may be passed when the anchor is in whole or in part within a joint or other subcutaneous portion of a patient's body. Any other effective structure, which may or may not include use of a suture, may be used to enable coupling of a soft tissue graft to an anchor in other embodiments. An alternate embodiment of an anchor may include a fork near a distal end of the anchor instead of one or more anchor suture holes, such as the anchor suture holes 106. In such an alternate embodiment, the fork may be used to capture a portion of an anchor suture, a portion of a soft tissue graft, a knot on a whipstitch between an anchor suture and a soft tissue graft, or any other effective element. Embodiments of such a fork may also include spikes, teeth, or other binding mechanisms to stop movement of a suture or soft tissue graft relative to the fork.

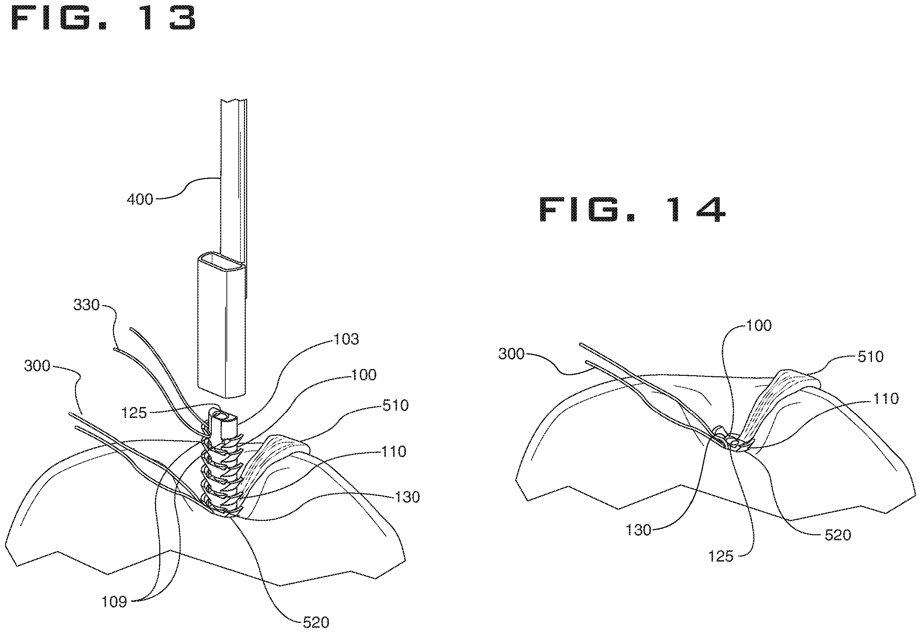

As shown in FIG. 13, the anchoring system 1 may also include the anchor length reduction tool 400. The anchor length reduction tool 400 illustrated is configured to fit over a proximal end 103 of the anchor 100 and be used to apply a force to the anchor 100 to reduce the length of the anchor 100. The anchor length reduction tool 400 illustrated may be pushed as far distally as desired by a user before applying a force to reduce the length of the anchor 100. In other embodiments, the distal insertion depth of an anchor length reduction tool may be limited relative to the anchor by components of one or both of the anchor length reduction tool and the anchor.

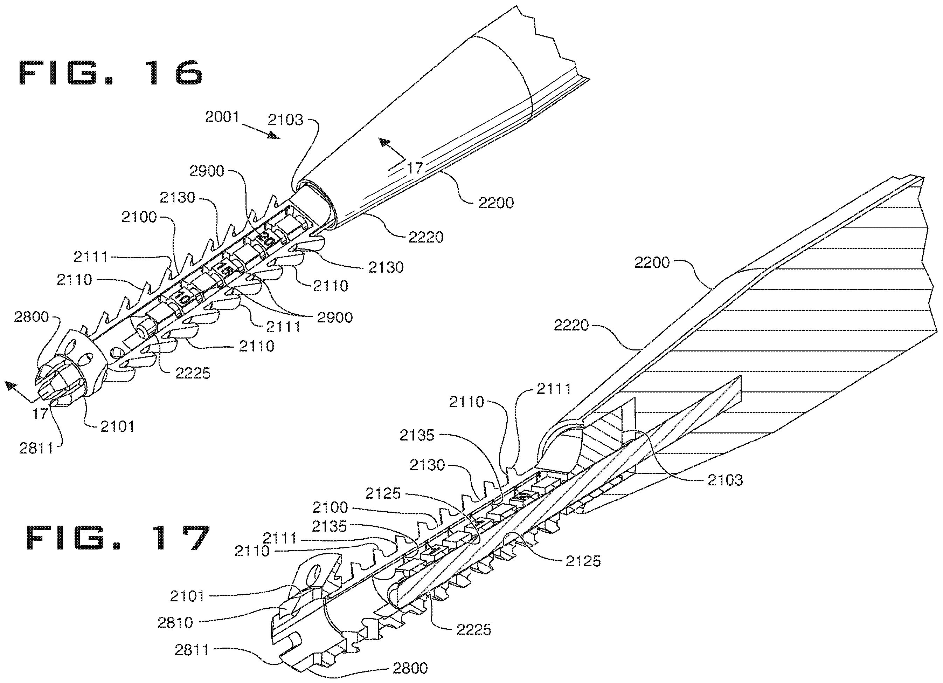

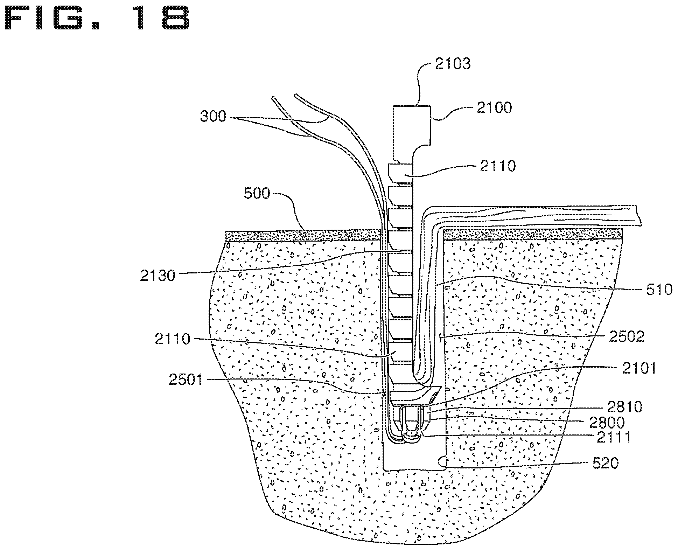

An embodiment of an anchoring system 2001 and its component parts and methods of implementation are illustrated in FIGS. 16-18. As shown in FIGS. 16 and 17, the anchoring system 2001 may include an anchor 2100 and an inserter 2200. Various features of the anchor 2100 are illustrated in combination in FIGS. 16-18. The anchor 2100 shown includes an anchor body with a distal end 2101, a proximal end 2103, and a length between the distal end 2101 and the proximal end 2103. The illustrated embodiment also shows anti-backout features in the form of teeth along the length of the anchor body. The teeth 2110 shown are configured to resist proximal movement of the anchor 2100 when the anchor 2100 has been inserted in a hole 520 (FIG. 18) in a bone 500 with a diameter equal to or less than a maximum diameter of the anchor 2100, as the term "diameter" has been defined previously herein.

Embodiments of the invention may include an anchor body with a cross-sectional shape that has a larger diameter in a first direction and a relatively smaller diameter in a second direction. For example and without limitation, the anchor 2100 has a larger diameter illustrated laterally across the dimension on which numbers 2900 are displayed in FIG. 16 and a smaller diameter illustrated laterally across FIG. 18. The smaller diameter may be transverse to the first direction such that when an anchor, such as the anchor 2100, is inserted in a hole that is substantially the same diameter as the larger diameter, a first space exists in the direction of a first side of the smaller diameter through which a suture may be passed and a second space exists in a direction of a second side of the smaller diameter through which a soft tissue graft coupled to the suture may be passed. For example, as depicted in FIG. 18, a first space 2501 exists in the direction of a back side of the anchor 2100 through which the anchor suture 300 may be passed. A second space 2502 exists in the direction of a front side of the anchor 2100 through which the soft tissue graft 510 may be passed. With the anchor 2100 in the position illustrated in FIG. 18, the larger diameter of the anchor 2100 is engaged with a wall of the hole 520 to secure the anchor 2100 relative to the bone 500. The second, transverse direction of the illustrated embodiment is substantially perpendicular to the first direction. However, in other embodiments the directions may not be substantially perpendicular but are transverse to some degree. The distal end 2101 of the anchor 2100 includes a portion that is substantially round with a diameter in all directions larger than the smaller diameter of the anchor 2100. Other embodiments may or may not include a substantially round distal end portion. As used herein, a hole substantially the same diameter as the larger diameter is not considered to be an exact specification, particularly in light of the fact that holes in bone may be relatively imprecise or irregular.

The teeth 2110 depicted may be resilient or flexible such that the teeth 2110 flex away from the wall of the hole 520 (FIG. 18) when the anchor 2100 is inserted into the hole 520. A resilient or flexible tooth may also include an angular edge, such as the angular edge 2111, that catches against the wall of the hole 520 if a proximal force is applied to the anchor 2100. Anti-backout features of these or other embodiments may include barbs, hooks, spikes, or any other effective mechanism to resist backout of an anchor to which the features are attached. For example and without limitation, anti-backout elements may be triangular, square, pyramid shaped, shark's tooth shaped, and raked to any effective degree.

The anchor body of the anchor 2100 shown is segmented at multiple locations along its length such that the length of the anchor 2100 may be reduced by removing the proximal end 2103 and a portion of the length of the anchor body. Segmentations at multiple locations along the anchor body of the anchor 2100 are illustrated in FIGS. 16-18. The segmentations shown include zones of reduced material thickness 2130 that are configured to be fractured to reduce the length of the anchor body of the anchor 2100. Zones configured to be fractured may be cross-sectional portions of an anchor that have less material volume compared with other cross-sections of the anchor or may have features specifically designed to fracture more readily. As particularly well illustrated in FIG. 17, the zones of reduced material thickness 2130 may include scored lines 2135 that further reduce material thickness at specific locations where fractures may be induced. The scored lines 2135 may also assist with breaking the anchor 2100 more cleanly along a specific path. These or similar scorings, etchings, molded indents or the like, may be employed with various embodiments. The portion of the anchor 2100, or other anchor embodiments, to be removed may be of any useful length, and fracture locations generally but not necessarily align with zones of reduced material thickness. For example and without limitation, a fracture location of some embodiments could be dictated by the placement and configuration of a cooperating anchor length reduction tool. The zones of reduced material thickness 2130 of the illustrated embodiment may also provide through-growth holes for tissue to penetrate and grow through the anchor 2100 and thereby enhance fixation of the anchor 2100 over time. Through-growth holes may be included in other embodiments that are not part of zones of reduced material thickness. The anchor 1100 and the anchor 2100 are segmented by couplings between snap together components of any effective type. For example and without limitation, couplings between snap together components may include adhesives, some frangible material segment, interdigitating components, or may merely be fixed together relatively firmly and held by friction.

The embodiment illustrated in FIGS. 16-18 also includes a trap 2800 through the anchor body near the distal end 2101 of the anchor body through which a portion of a soft tissue graft 510 (FIG. 18) may be passed. The trap 2800 illustrated resists a reverse passage of the soft tissue graft 510 from the trap 2800. The trap 2800 is composed of several resilient fingers 2810 that include an angular edge 2811 that are configured to catch against the soft tissue graft 510 if a proximal force is applied to the soft tissue graft 510 relative to the anchor 2100. Any other mechanism that permits movement of a soft tissue graft through a hole in one direction, but resists movement in an opposite direction may be used in other embodiments. As illustrated in FIG. 18, the anchor suture 300 and the soft tissue graft 510 may be passed through the trap 2800 together. The anchor suture 300 may be pulled proximally to advance the soft tissue graft 510 further distally. The anchor suture 300 of some embodiments may also be tied off relative to the anchor 2100 or the inserter 2200 to help secure, whether temporarily or permanently, the soft tissue graft 510 relative to the anchor 2100. Some trap embodiments may be sized such that an anchor suture, such as the anchor suture 300 (rather than a soft tissue graft), may be allowed to pass in one direction through a trap that resists movement of the suture in an opposite direction.

The anchor body of the anchor 2100 includes numbers 2900 (FIG. 16) to provide an indication of a depth of penetration of the anchor 2100 into the hole 520 (FIG. 18). The numbers 2900 illustrated are "10", "15", and "20". These numbers indicated that when the particular number is visible at the surface of a bone in which the anchor 2100 is being inserted, that a far distal tip of the anchor 2100 has penetrated into the bone the least number of millimeters visible. In this example, the numbers shown would represent 10mm, 15mm, or 20mm. Interpolations between the numbers shown would also be available to a user of the anchor. In some embodiments, a user may be able to determine depth of penetration by counting the number of teeth 2110 visible above the surface of a bone in which an anchor 2100 is being inserted. For example and without limitation, the distance between each tooth may be 2mm. If it were know that the distance from the angular edge 2111 of the most proximal tooth to the far distal tip of the anchor was, for example 24mm, then a depth of penetration could be determined by multiplying the number of teeth exposed by 2mm and subtracting that number from 24mm. These specific values are only exemplary and other embodiments may use any appropriate dimensions.

Some features of the inserter 2200 are shown in combination in FIGS. 16 and 17. A distal end of the inserter 2200 is illustrated in FIGS. 16 and 17. The inserter 2200 may include a handle essentially similar to the handle 210 described herein, and features of such a handle will not be describe further here. A distal end of a shaft 2220 that may be coupled to a handle is shown in FIGS. 16 and 17. The shaft 2220 includes a connecting mechanism configured to releasably couple with the anchor 2100. In particular, the connecting mechanism illustrated includes a distal end 2225 of the shaft 2220 that extends along and contacts a portion of the anchor 2100 to support the anchor 2100 (FIGS. 16 and 17). In some embodiments, such support provides a temporary substantially rigid construct for insertion. The portion of the anchor 2100 contacted is a cannulation 2125 (FIG. 17) in the illustrated embodiment. However, in other embodiments other portions of an anchor may be supported by portions of an inserter to facilitate insertion of an anchor such as the segmented anchor 2100 that may benefit from additional support. As shown in FIG. 17, the proximal end 2103 of the anchor 2100 also fits within a portion of the shaft 2220 of the inserter 2200 to form a part of the connecting mechanism of the inserter 2200 in cooperation with the anchor 2100. In the particular case of the illustrated embodiment, the anchor 2100 benefits from additional support of the distal end 2225 of the shaft 2220 while insertion forces are being applied to the inserter and anchor construct. After the anchor 2100 is inserted, the inserter 2200, and particularly the distal end 2225 of the shaft 2220, may be removed from the anchor 2100, as depicted in FIG. 18.

The connecting mechanism of the inserter 2200 in cooperation with the anchor 2100 includes friction between the distal end 2225 of the shaft 2220 and the cannulation 2125 and between the proximal end 2103 of the anchor 2100 and the shaft 2220 of the inserter 2200. In other embodiments, any other effective connecting mechanism may be used in addition or alternatively to removably couple between an inserter and an anchor of this or other embodiments. For example and without limitation, an anchor of some embodiments may be held to an inserter with a stay suture or by some mechanical feature such as a snap, hook, or ball and detent, rather than or in addition to a stay suture.

The anchoring system 2001 may also include an anchor length reduction tool essentially similar to the anchor length reduction tool 400 described herein, or another tool that provides similar functionality.

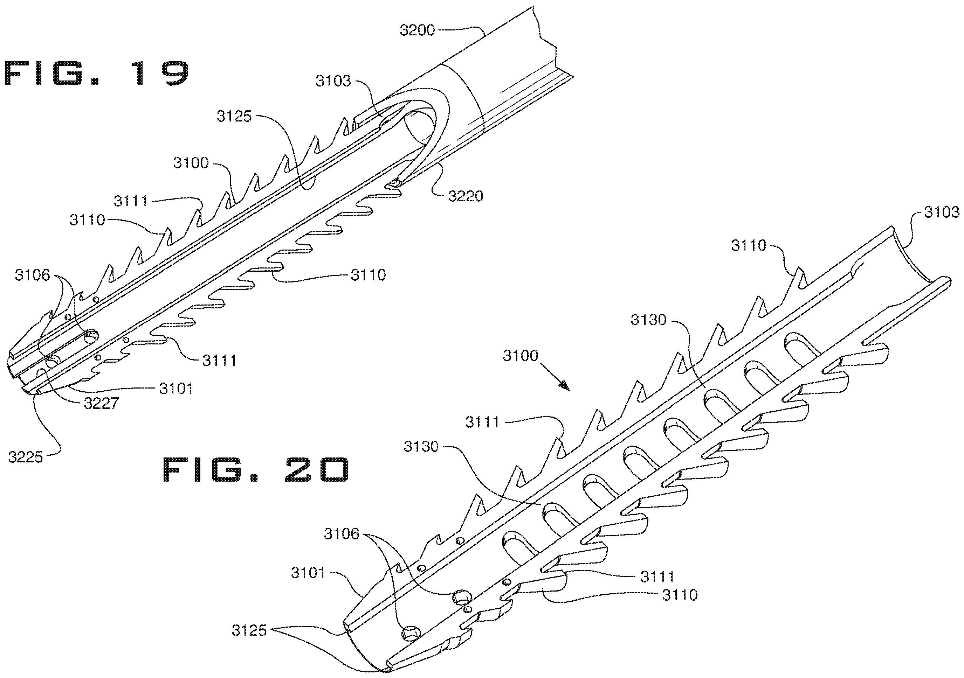

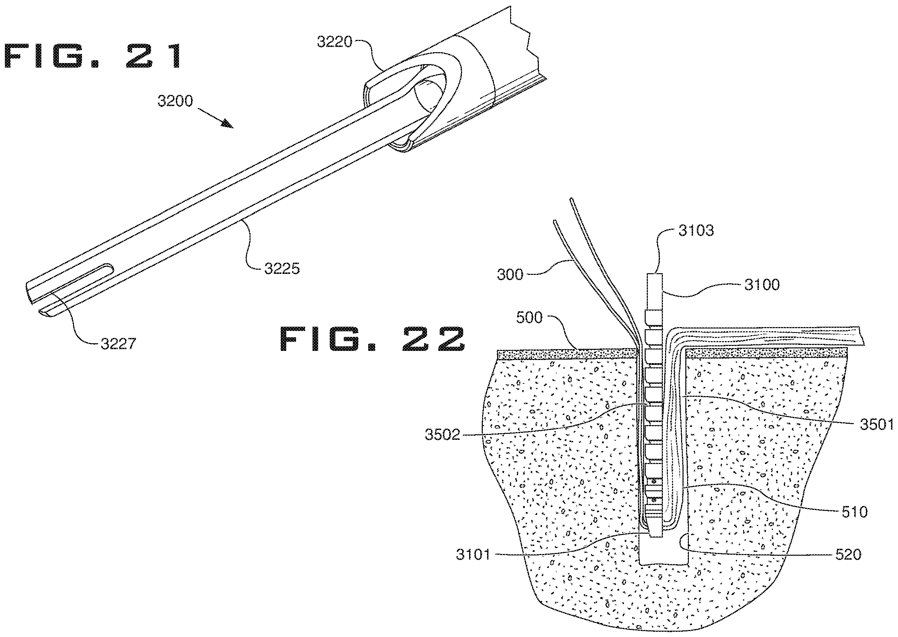

An embodiment of an anchoring system 3001 and its component parts and methods of implementation are illustrated in FIGS. 19-22. As shown in FIG. 19, the anchoring system 3001 may include an anchor 3100 and an inserter 3200. Various features of the anchor 3100 are illustrated in combination in FIGS. 19, 20, and 22. The anchor 3100 shown includes an anchor body with a distal end 3101, a proximal end 3103, and a length between the distal end 3101 and the proximal end 3103. The illustrated embodiment also shows anti-backout features in the form of teeth 3110 along the length of the anchor body. The teeth 3110 shown are configured to resist proximal movement of the anchor 3100 when the anchor 3 100 has been inserted in a hole 520 (FIG. 22) in a bone 500 with a diameter equal to or less than a maximum diameter of the anchor 3100, as the term "diameter" has been defined previously herein.

Embodiments of the invention may include an anchor body with a cross-sectional shape that has a larger diameter in a first direction and a relatively smaller diameter in a second direction. For example and without limitation, the anchor 3100 has a larger diameter illustrated laterally across the dominant side dimension illustrated in FIGS. 19 and 20 and a smaller diameter illustrated laterally across FIG. 22. The smaller diameter may be transverse to the first direction such that when an anchor, such as the anchor 3100, is inserted in a hole that is substantially the same diameter as the larger diameter, a first space exists in the direction of a first side of the smaller diameter through which a suture may be passed and a second space exists in a direction of a second side of the smaller diameter through which a soft tissue graft coupled to the suture may be passed. For example, as depicted in FIG. 22, a first space 3502 exists in the direction of a back side of the anchor 3100 through which the anchor suture 300 may be passed. A second space 3501 exists in the direction of a front side of the anchor 3100 through which the soft tissue graft 510 may be passed. With the anchor 3100 in the position illustrated in FIG. 22, the larger diameter of the anchor 3100 is engaged with a wall of the hole 520 to secure the anchor 3100 relative to the bone 500. The second, transverse direction of the illustrated embodiment is substantially perpendicular to the first direction. However, in other embodiments the directions may not be substantially perpendicular but are transverse to some degree.

The teeth 3110 depicted may be resilient or flexible such that the teeth 3110 flex away from the wall of the hole 520 (FIG. 22) when the anchor 3100 is inserted into the hole 520. A resilient or flexible tooth may also include an angular edge, such as the angular edge 3111, that catches against the wall of the hole 520 if a proximal force is applied to the anchor 3100. Anti-backout features of these or other embodiments may include barbs, hooks, spikes, or any other effective mechanism to resist backout of an anchor to which the features are attached. For example and without limitation, anti-backout elements may be triangular, square, pyramid shaped, shark's tooth shaped, and raked to any effective degree.

The anchor body of the anchor 3100 shown is segmented at multiple locations along its length such that the length of the anchor 3100 may be reduced by removing the proximal end 3103 and a portion of the length of the anchor body. Segmentations at multiple locations along the anchor body of the anchor 3100 are illustrated in FIG. 20. The segmentations shown include zones of reduced material thickness 3130 that are configured to be fractured to reduce the length of the anchor body of the anchor 3100. Zones configured to be fractured may be cross-sectional portions of an anchor that have less material volume compared with other cross-sections of the anchor or may have features specifically designed to fracture more readily. Zones of reduced material thickness 3130 may include scored lines that further reduce material thickness at specific locations where fractures may be induced or may assist with breaking the anchor 3100 more cleanly along a specific path. One or more scorings, etchings, molded indents or the like, may be employed with various embodiments. The portion of the anchor 3100, or other anchor embodiments, to be removed may be of any useful length, and fracture locations generally but not necessarily align with zones of reduced material thickness. For example and without limitation, a fracture location of some embodiments could be dictated by the placement and configuration of a cooperating anchor length reduction tool. The zones of reduced material thickness 3130 of the illustrated embodiment may also provide through-growth holes for tissue to penetrate and grow through the anchor 3100 and thereby enhance fixation of the anchor 3100 over time. Through-growth holes may be included in other embodiments that are not part of zones of reduced material thickness. The anchor 1100 and the anchor 3100 are segmented by couplings between snap together components of any effective type. For example and without limitation, couplings between snap together components may include adhesives, some frangible material segment, interdigitating components, or may merely be fixed together relatively firmly and held by friction.

The anchor body of the anchor 3100 may include numbers to provide an indication of a depth of penetration of the anchor into the hole 520, as described in association with the anchor 2100 herein. Also similar to the embodiment of anchor 2100, depth of penetration may be determined based on numbers of teeth extending above the surface of a bone during a given state of insertion.

Some features of the inserter 3200 are shown in combination in FIGS. 19 and 21. A distal end of the inserter 3200 is illustrated in FIGS. 19 and 21. The inserter 3200 may include a handle essentially similar to the handle 210 described herein, and features of such a handle will not be describe further here. A distal end of a shaft 3220 that may be coupled to a handle is shown in FIGS. 19 and 21. The shaft 3220 includes a connecting mechanism configured to releasably couple with the anchor 3100. In particular, the connecting mechanism illustrated includes a distal end 3225 of the shaft 3220 that extends along and contacts a portion of the anchor 3100 to support the anchor 3100 (FIG. 19). In some embodiments, such support provides a temporary substantially rigid construct for insertion. The portion of the anchor 3100 contacted in the illustrated embodiment is a set of channels 3125 (FIGS. 19 and 20). However, in other embodiments other portions of an anchor may be supported by portions of an inserter to facilitate insertion of an anchor such as the segmented anchor 3100 that may benefit from additional support. In the particular case of the illustrated embodiment, the anchor 3100 benefits from additional support of the distal end 3225 of the shaft 3220 while insertion forces are being applied to the inserter and anchor construct. As illustrated in FIG. 19, the distal end 3225 of the shaft 3220 of some embodiments is sized to extend to a far distal end of the anchor 3100 when the anchor 3100 is in position on the inserter 3200 to be inserted in the hole 520. This configuration provides for support along the entire length of the anchor body of the anchor 3100. In other embodiments, the distal end of a shaft of an inserter may be shorter relative to an anchor, but may still provide an important support function. For example, some embodiments may provide for support along a majority of an anchor, although the support is not provided to a far distal end. Still other embodiments may provide for even a shorter support by an inserter. After the anchor 3100 of the illustrated embodiment is inserted, the inserter 3200, and particularly the distal end 3225 of the inserter 3200, may be removed from the anchor 3100, as depicted in FIG. 22.

The connecting mechanism of the inserter 3200 in cooperation with the anchor 3100 includes friction between the distal end 3225 of the inserter 3200 and the channels 3125. In other embodiments, any other effective connecting mechanism may be used in addition or alternatively to removably couple between an inserter and an anchor of this or other embodiments. For example and without limitation, an anchor of some embodiments may be held to an inserter with a stay suture or by some mechanical feature such as a snap, hook, or ball and detent, rather than or in addition to a stay suture.

In FIG. 22, the anchor suture 300 has been whipstitched to the soft tissue graft 510. The anchor suture 300 illustrated includes two strands of anchor suture 300 that may be threaded through suture holes 3106 (FIGS. 19 and 20) and connected with an anchor suture holder similar to the anchor suture holder 240 (FIG. 11). In FIG. 22, the anchor suture 300 has been pulled proximally until the soft tissue graft 510 has been moved as far distally as possible for this embodiment. This distal movement may occur before, during, or after insertion of the anchor 3100 into the bone 500. The distal end 3225 of the inserter 3200 includes a notch 3227 (FIGS. 19 and 21) that aligns with the suture holes 3106 so that the inserter 3200 may be move onto or removed from the anchor 3100 even when the anchor suture 300 has been passed through the suture hole 3106. In other embodiments, graft tissue, a patient's tissue, or sutures of any type, or a combination of such elements may be passed through an opening such as one or both of the suture holes 3106 for anchoring. Sutures of the anchoring system may be any type of suture, for example and without limitation, a monofilament, multistrand, or woven construct. A suture may be passed through an anchor while the anchor is outside of a patient's body, or may be passed when the anchor is in whole or in part within a joint or other subcutaneous portion of a patient's body. Any other effective structure, which may or may not include use of a soft tissue graft, may be used to enable coupling of a suture to an anchor in other embodiments.

The anchoring system 3001 may also include an anchor length reduction tool essentially similar to the anchor length reduction tool 400 described herein, or another tool that provides similar functionality.

Any of the anchors 100, 1100, 2100, 3100, as well as other anchor embodiments, may be referred to as frangible anchors. As used herein, "frangible" refers to an anchor that breaks in a predictable and desirable location when subjected to a sufficient force.

An example of the disclosure is a method of inserting a soft tissue graft, such as soft tissue graft 510, into a bone, such as illustrated bone 500. A suture, such as the anchor suture 300 (FIGS. 11-14, 18, and 22) is provided for coupling to the soft tissue graft 510 for the illustrated embodiments. Some embodiments may include passing the suture around a portion of an anchor. For example, in the embodiments depicted, the anchor suture 300 is passed around a portion of the anchor 100, 1100, 2100, 3100 near the distal end 101, 2101, 3101 of the anchor 100, 2100, 3100 by passing the anchor suture 300 through the suture holes 106, 3106 (trap 2800). Any other effective structure, which may or may not include use of a suture, may be used to enable coupling of a soft tissue graft to an anchor in other embodiments. In the embodiment illustrated in FIGS. 16-18, the method may include passing the soft tissue graft through the trap 2800 near the distal end 2101 of the anchor body 2100. The trap 2800 resists a reverse passage of the soft tissue graft 510 from the trap 2800. A "reverse passage" as used herein means movement in a directions substantially opposite from the direction of initial insertion.

The example anchors 100, 1100, 2100, 3100 include a body with a cross-sectional shape that has a larger diameter in first direction and a relatively smaller diameter in a second direction that is transverse to the first direction. When the anchor 100, 1100, 2100, 3100 is inserted in a hole 520 in the bone 500 substantially the same diameter as the larger diameter, a first space 501, 2501, 3502 exists in a direction of a first side of the smaller diameter through which an anchor suture 300 may be passed and a second space 502, 2502, 3501 exists in a direction of a second side of the smaller diameter through which a soft tissue graft 510 coupled to the anchor suture 300 may be passed. Method embodiments may also include inserting an anchor, such as the anchor 100, 1100, 2100, 3100, into the bone 500 such that the larger diameter of the anchor 100, 1100, 2100, 3100 firmly engages with the bone 500 until the soft tissue graft 510 being advanced with the anchor 100, 1100, 2100, 3100 is tensioned to a desirable degree. The anchor suture 300 may be tied near the suture holes 106, 3106 (trap 2800) in some embodiments. In other embodiments, the anchor suture 300 may not be initially fixed so that the position of the soft tissue graft 510 may be adjusted throughout the procedure. For example, an anchor, such as the anchor 100, 1100, 2100, 3100 may be pushed distally as far as desired, after which the anchor suture 300 may be pulled distally to move the soft tissue graft 510 into a desired position relative to the anchor 100, 1100, 2100, 3100. An additional advantage of some embodiments of the present invention, as evidenced here, is that an anchor may be pushed longitudinally into tissue such as bone without twisting. A longitudinal insertion path may assist a surgeon in protecting the soft tissue graft and sutures from damage and may lead to more precisely adjustable soft tissue graft tensioning.

Still another example of the disclosure is a method of inserting an anchor, such as the anchor 100, 1100, 2100, 3100, into a bone, such as the bone 500. As an example, the method may include coupling the anchor 100, 1100, 2100, 3100 and an inserter 200, 2200, 3200 by placing a distal end 225, 2225, 3225 connecting mechanism of the inserter 200, 2200, 3200 into a cooperating portion of the anchor 100, 1100, 2100, 3100 to provide support to the anchor 100, 1100, 2100, 3100, as particularly well illustrated in FIGS. 8, 9, 16, 17, and 19. The connecting mechanism of embodiments of FIGS. 8, 9, 16, and 17 work with the cannulation 125, 2125 in the anchor 100, 2100. The connecting mechanism of the embodiment of FIG. 19 works with the channels 3125. Other embodiments may only include a single channel and may include channels of other cross-sectional shapes. Anchors of various embodiments may or may not have a suture or soft tissue graft attached throughout the procedure described in association with this method. For example and without limitation, in some methods only one or more anchor sutures 300 that are connected to a soft tissue graft 510 may be inserted through the anchor 100, 1100, 2100, 3100 until after the anchor 100, 1100, 2100, 3100 is implanted in the bone 500. After the anchor 100, 1100, 2100, 3100 of such embodiments is in place, then a distal end of the anchor suture 300 may be pulled proximally to advance the soft tissue graft 510 distally relative to the anchor 100, 1100, 2100, 3100. In the embodiment illustrated in FIG. 11, coupling between the anchor 100 and the inserter 200 includes attaching a stay suture 330 between the inserter 200 and the anchor 100. In particular, two strands of the stay suture 330 are routed through the stay suture holes 109 (FIGS. 2, 4, 6, 8, 9, and 13) and removably coupled to the stay suture holders 230.

Method examples may include inserting an anchor, such as the anchor 100, 1100, 2100, 3100, into bone, such as the bone 500. Such insertion may include inserting an anchor into a preformed hole in bone, such as the hole 520 shown in FIGS. 12, 18, and 22, or may include forcing a self-penetrating anchor into a bone or other tissue. An inserter may then be decoupled from an anchor in a further act of some embodiments.

Some method examples include inserting a frangible anchor, or anchor that may be broken by any mechanism described herein, fully within a bone without breaking the anchor. In other words, because the anchor is configured to be fractured or is otherwise frangible, does not mean that use of the anchor must include fracturing the anchor in all applications. In some embodiments, it may be advantageous to have an anchor configured to be fractured that is not required to be fractured in all applications. Such a capability enables the implant to provide a solution when a longer anchor is called for to meet a clinical need.

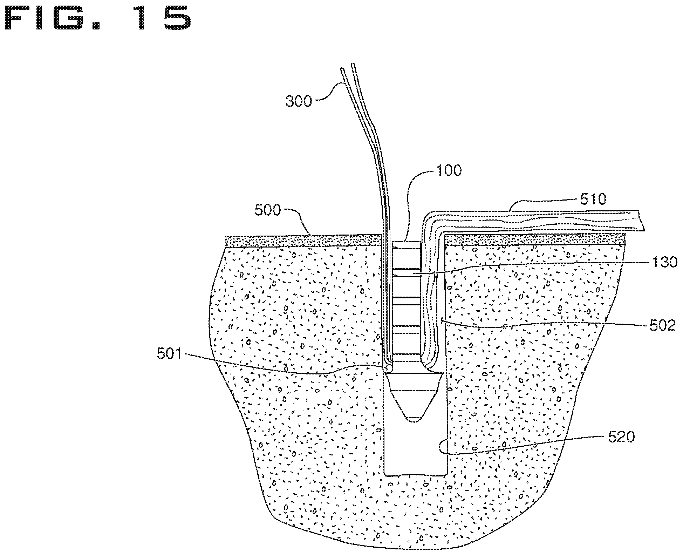

Method examples may also include breaking off a proximal end, such as the proximal end 103, 2103, 3103, and a portion of a length of the anchor body. Breaking off a proximal end of some embodiments includes applying a force to the anchor to reduce the length of the anchor. For example, as shown in FIG. 13, the anchor length reduction tool 400 may be moved longitudinally over the proximal end 103, 2103, 3103 of the anchor 100, 1100, 2100, 3100 and a force may be applied to the anchor 100, 1100, 2100, 3100 to break off the anchor 100, 1100, 2100, 3100 at a desired location. For example, a moment force may be applied by moving a proximal end of the anchor length reduction tool 400 away from a longitudinal axis of the anchor 100. Breaking off of an anchor of some embodiments could also include applying a shear force (such as cutting with a cutting tool), a tensile force, a twisting force, or any other effective combination of these or other forces. In some embodiments, such a force may be applied without a specialized tool such as the anchor length reduction tool 400 or without any tool. As illustrated to varying degrees in FIG. 14 (slightly above bone surface) and FIG. 15 (slightly below bone surface), some embodiments include breaking an anchor 100, 1100, 2100, 3100 off substantially flush with a surface of a bone 500.

The present invention relates to a frangible anchor including at least an anchor body with a distal end, a proximal end, and a length between the distal end and the proximal end, wherein the anchor body is segmented at one or more locations along its length such that the length of the frangible anchor may be reduced by removing the proximal end and a portion of the length of the anchor body. The anchor body may be segmented by one or more zones of reduced material thickness that are configured to be fractured to reduce the length of the anchor body. The anchor body is segmented by one or more couplings between snap together components, wherein the snap together components are configured to be detached at the one or more couplings to reduce the length of the anchor body. The anchor body may include one or more anti-backout features along the length of the anchor body configured to resist proximal movement of the frangible anchor when the frangible anchor has been inserted in a hole with a diameter equal to or less than a maximum diameter of the frangible anchor. The one or more anti-backout features may include a tooth. The tooth may be resilient and be configured to be flexed away from a wall of the hole when the anchor is inserted into the hole, and the flexible tooth may include an angular edge that catches against the wall of the hole if a proximal force is applied to the frangible anchor. The one or more anti-backout features may include a barb. The frangible anchor may include a trap through the anchor body near the distal end of the anchor body through which a portion of a soft tissue graft may be passed, wherein the trap resists a reverse passage of the soft tissue graft from the trap. The anchor body may include one or more numbers marked on the anchor body to provide an indication of a depth of penetration of the anchor into the hole. The anchor body may have a cannulation to receive a distal portion of an inserter to strengthen the anchor body during insertion. The anchor body may include a channel along a portion of the length of the anchor body in which a portion of a distal end of an inserter is sized to fit to strengthen the anchor body during insertion. The channel may extend along a majority of the length of the anchor body.

An example of the disclosure includes at least an anchor having an anchor body with a distal end, a proximal end, and a length between the distal end and the proximal end; wherein the anchor body includes a cross-sectional shape that has a larger diameter in a first direction and a relatively smaller diameter in a second direction that is transverse to the first direction such that when the anchor is inserted in a hole substantially the same diameter as the larger diameter a first space exists in a direction of a first side of the smaller diameter through which a suture may be passed and a second space exists in a direction of a second side of the smaller diameter through which a soft tissue graft coupled to the suture may be passed; and wherein the anchor body is segmented at one or more locations along its length such that the length of the anchor may be reduced by removing the proximal end and a portion of the length of the anchor body. The anchor body may include a hole through the anchor body near the distal end of the anchor body through which the suture may be passed. The anchor body may include a pocket adjacent to the hole through the anchor body near the distal end of the anchor body sized to allow the soft tissue graft to be passed into the pocket. The anchor body may include a trap through the anchor body near the distal end of the anchor body through which a portion of a soft tissue graft may be passed, wherein the trap resists a reverse passage of the soft tissue graft from the trap. The anchor body may include one or more numbers marked on the anchor body to provide an indication of a depth of penetration of the anchor into the hole. A portion of the distal end of anchor may be substantially round with a diameter in all directions larger than the smaller diameter. The anchor body may include one or more anti-backout features along the length of the anchor body in the direction of the larger diameter configured to resist proximal movement of the anchor when the anchor has been inserted in an anchor hole with a diameter equal to or less than the larger diameter of the anchor body. The one or more anti-backout features may include a tooth. The tooth may be resilient and be configured to be flexed away from a wall of the anchor hole when the anchor is inserted into the anchor hole, and the resilient tooth may include an angular edge that catches against the wall of the anchor hole if a proximal force is applied to the anchor. The one or more anti-backout features may include a barb. The anchor body may include a cannulation to receive a distal portion of an inserter to strengthen the anchor body during insertion. The anchor body may include a channel along a portion of the length of the anchor body in which a portion of a distal end of an inserter is sized to fit to strengthen the anchor body during insertion. The channel may extend along a majority of the length of the anchor body. The anchor body may be segmented by one or more zones of reduced material thickness that are configured to be fractured to reduce the length of the anchor body. The anchor body is segmented by one or more couplings between snap together components, wherein the snap together components are configured to be detached at the one or more couplings to reduce the length of the anchor body.

An example of the disclosure is a method of inserting a soft tissue graft into a bone including at least the acts of providing a suture for coupling to the soft tissue graft; passing the suture around a portion of an anchor wherein the anchor includes a body with a cross-sectional shape that has a larger diameter in first direction and a relatively smaller diameter in a second direction that is transverse to the first direction such that when the anchor is inserted in a hole in the bone substantially the same diameter as the larger diameter a first space exists in a direction of a first side of the smaller diameter through which a suture may be passed and a second space exists in a direction of a second side of the smaller diameter through which a soft tissue graft coupled to the suture may be passed; and inserting the anchor into the bone such that the larger diameter of the anchor firmly engages with the bone and until the soft tissue graft being advanced with the anchor is tensioned to a desirable degree. The act of passing the suture around a portion of the anchor may include passing the suture through a hole through the anchor. The act of passing the suture around a portion of the anchor may include passing the suture around a portion of the anchor near a distal end of the anchor. The act of passing the soft tissue graft through a trap through the anchor body near the distal end of the anchor body may include the trap resisting a reverse passage of the soft tissue graft from the trap. The method may also include the act of breaking off a proximal end and a portion of a length of the anchor body. The act of breaking off a proximal end and a portion of a length of the anchor body may include applying a force to the anchor to reduce the length of the anchor. The act of applying a force may include applying a moment force. The act of breaking off a proximal end and a portion of a length of the anchor body may include breaking the anchor body off substantially flush with a surface of the bone.

An example of the disclosure is a method of inserting an anchor into a bone including at least coupling the anchor and an inserter by placing a distal end connecting mechanism of the inserter into a cooperating portion in the anchor to provide support to the anchor; inserting the anchor into the bone; decoupling the inserter and the anchor; and breaking off a proximal end and a portion of a length of the anchor. The act of coupling the anchor and the inserter may include attaching a stay suture between the inserter and the anchor. The act of coupling the anchor and the inserter may include placing a connecting mechanism of the inserter into a cooperating portion in the anchor that is a cannulation in the anchor. The act of coupling the anchor and the inserter may include placing a connecting mechanism of the inserter into a cooperating portion in the anchor that is a channel in the anchor. The act of inserting the anchor into the bone may include inserting the anchor into a preformed hole in the bone. The act of inserting the anchor into the bone may include inserting the anchor into the bone where the anchor is self-penetrating. The act of breaking off a proximal end and a portion of a length of the anchor body may include applying a force to the anchor to reduce the length of the anchor. The act of applying a force may include applying a moment force. The act of breaking off a proximal end and a portion of a length of the anchor body may include breaking the anchor body off substantially flush with a surface of the bone.

Various embodiments of a system wholly or its components individually may be made from any biocompatible material. For example and without limitation, materials may include in whole or in part: non-reinforced polymers, reinforced polymers, metals, ceramics, adhesives, reinforced adhesives, and combinations of these materials. Reinforcing of polymers may be accomplished with carbon, metal, or glass or any other effective material. Examples of biocompatible polymer materials include polyamide base resins, polyethylene, Ultra High Molecular Weight (UHMW) polyethylene, low density polyethylene, polymethylmethacrylate (PMMA), polyetheretherketone (PEEK), polyetherketoneketone (PEKK), a polymeric hydroxyethylmethacrylate (PHEMA), and polyurethane, any of which may be reinforced. Example biocompatible metals include stainless steel and other steel alloys, cobalt chrome alloys, zirconium, oxidized zirconium, tantalum, titanium, titanium alloys, titanium-nickel alloys such as Nitinol and other superelastic or shape-memory metal alloys. The material of some embodiments of the anchor or its components may include a resorbable material, which over time is incorporated into a patient's tissue in which the device is implanted. Effective resorbable materials of some embodiments provide an adequate level of strength and stiffness for a time frame that exceeds that expected for tissue healing. After healing, such devices may be completely or otherwise significantly absorbed by a patient's body. The biological performance for some embodiments, particularly for application at a bony site, may be enhanced by incorporation of bioactive fillers in the polymer. A non-limiting list of synthetic and natural biodegradable polymers includes polyglycolide, polyhydroxobutyrate, chitosan, hyaluronic acid, and hydrogels. Poly(2-hydroxyethylmethacrylate) and poly(ethylene glycol) may be used, as may poly(L-lactide) (PLA). REGENESORB is a trade named resorbable polymer material that may be used and includes D,L-polylactid acid. Embodiments of the anchor may be made via a molding process or other process known to one of skill in the art. The internal and external surfaces of embodiments of the distal anchor component may be formed during a molding process or may be formed by drilling or machining. Sutures or other similar components of the invention may be single strand, woven, braided, or any combination thereof from any of these or other biocompatible materials. The sutures or other similar components may be any effective natural or synthetic material and may be a use or combination of materials well-known in the art. Sutures or other similar components of various embodiments may be resorbable or not resorbable.

Terms such as proximal, distal, far, along, near, over, and the like have been used relatively herein. However, such terms are not limited to specific coordinate orientations, distances, or sizes, but are used to describe relative positions referencing particular embodiments. Such terms are not generally limiting to the scope of the claims made herein. Any embodiment or feature of any section, portion, or any other component shown or particularly described in relation to various embodiments of similar sections, portions, or components herein may be interchangeably applied to any other similar embodiment or feature shown or described herein.

While embodiments of the invention have been illustrated and described in detail in the disclosure, the disclosure is to be considered as illustrative and not restrictive in character. All changes and modifications are to be considered within the scope of the disclosure.

## Claims

1. An anchoring system (1) comprising:
an anchor (100) comprising:
an anchor body with a distal end (101), a proximal end (103), and a length between the distal end and the proximal end, and
one or more anti-backout features (110) along the length of the anchor body configured to resist proximal movement of the anchor when the anchor has been inserted in a hole (520) with a diameter equal to or less than a maximum diameter of the anchor (100),
wherein the anchor body is segmented at one or more locations (130) along its length such that the length of the anchor (100) may be reduced by removing the proximal end (103) and a portion of the length of the anchor body; and
an inserter (200) comprising:
a handle (210), and
a shaft (220) coupled to the handle (210) wherein the shaft (220) includes a connecting mechanism configured to releasably couple with the anchor,
wherein the connecting mechanism includes a distal end (225) of the shaft (220) that extends along and contacts a portion of the anchor (100) to support the anchor (100);
wherein the anchor body of the anchor (100) has a cannulation (125);
wherein the distal end (225) of the shaft (220) is sized to fit within the cannulation (125) in the anchor (100) to provide support to the anchor (100); and
**characterised in that** the anchor body is segmented by one or more couplings (1130) between snap together components (1150), wherein the snap together components (1150) are configured to be detached at the one or more couplings (1130) to reduce the length of the anchor body.

2. The anchoring system of claim 1 wherein the one or more anti-backout features includes a tooth (110).

3. The anchoring system of claim 2 wherein the tooth (110) is resilient and is configured to be flexed away from a wall of the hole (520) when the anchor (100) is inserted into the hole (520), and wherein the flexible tooth (110) includes an angular edge (111) that catches against the wall of the hole (520) if a proximal force is applied to the anchor (100).

4. The anchoring system of claim 1 wherein the one or more anti-backout features includes a barb.

5. The anchoring system of any one of claims 1 to 4 wherein the anchor body is segmented by one or more zones of reduced material thickness (130) that are configured to be fractured to reduce the length of the anchor body (100).

6. The anchoring system of any preceding claim wherein the anchor (2100) includes a trap (2800) through the anchor body near the distal end (2101) of the anchor body through which a portion of a soft tissue graft (510) may be passed, wherein the trap (2800) resists a reverse passage of the soft tissue graft (510) from the trap (2800).

7. The anchoring system of any one of the preceding claims wherein the anchor body includes one or more numbers (2900) marked on the anchor body to provide an indication of a depth of penetration of the anchor (2100) into the hole (520).

8. The anchoring system of any one of claims 1 to 7 wherein the connecting mechanism includes a stay suture (330) capable of removably coupling between the inserter (200) and the anchor (100).

9. The anchoring system of any one of claims 1 to 7 wherein the distal end (225) of the shaft (220) is sized to extend to a far distal end of the anchor (100) when the anchor (100) is in position on the inserter (200) to be inserted in the hole (520).

10. The anchoring system of claim 1 wherein the connecting mechanism includes a friction fit between the inserter (200) and the anchor (100).

11. The anchoring system of any one of the preceding claims, further comprising an anchor length reduction tool (400) configured to fit over a proximal end (103) of the anchor (100) and be used to apply a force to the anchor (100) to reduce the length of the anchor (100).

12. The anchoring system of any one of the preceding claims, further comprising a suture (300) configured to be coupled to the anchor (100) and to be coupled to a soft tissue graft (510).

## Patentansprüche

1. Ein Ankersystem (1), das Folgendes beinhaltet:
einen Anker (100), der Folgendes beinhaltet:
einen Ankerkörper mit einem distalen Ende (101), einem proximalen Ende (103) und einer Länge zwischen dem distalen Ende und dem proximalen Ende, und
ein oder mehrere Anti-Backout-Vorrichtungen (110) entlang der Länge des Ankerkörpers, die konfiguriert sind, um einer proximalen Bewegung des Ankers zu widerstehen, wenn der Anker in ein Loch (520) mit einem Durchmesser gleich wie oder kleiner als ein maximaler Durchmesser des Ankers (100) eingeführt worden ist,
wobei der Ankerkörper an einer oder mehreren Stellen (130) entlang seiner Länge segmentiert ist, sodass die Länge des Ankers (100) durch Entfernen des proximalen Endes (103) und eines Teils der Länge des Ankerkörpers reduziert werden kann; und
eine Einführungshilfe (200), die Folgendes beinhaltet:
einen Griff (210), und
einen Schaft (220), der mit dem Griff (210) gekoppelt ist, wobei der Schaft (220) einen Verbindungsmechanismus umfasst, der konfiguriert ist, um lösbar mit dem Anker zu koppeln,
wobei der Verbindungsmechanismus ein distales Ende (225) des Schafts (220) umfasst, das sich entlang eines Teils des Ankers (100) erstreckt und diesen berührt, um den Anker (100) zu unterstützen;
wobei der Ankerkörper des Ankers (100) eine Kanülierung (125) aufweist;
wobei das distale Ende (225) des Schafts (220) so bemessen ist, dass es in die Kanülierung (125) in dem Anker (100) passt, um dem Anker (100) Unterstützung bereitzustellen; und
**dadurch gekennzeichnet, dass** der Ankerkörper durch eine oder mehrere Kopplungen (1130) zwischen zusammenschnappbaren Komponenten (1150) segmentiert ist, wobei die zusammenschnappbaren Komponenten (1150) konfiguriert sind, um an der einen oder den mehreren Kopplungen (1130) abgetrennt werden zu können, um die Länge des Ankerkörpers zu reduzieren.

2. Ankersystem gemäß Anspruch 1, wobei die eine oder mehreren Anti-Backout-Vorrichtungen einen Zahn umfassen (110).

3. Ankersystem gemäß Anspruch 2, wobei der Zahn (110) elastisch ist und konfiguriert ist, um von einer Wand des Lochs (520) weggebogen zu werden, wenn der Anker (100) in das Loch (520) eingeführt wird, und wobei der biegsame Zahn (110) eine winkelförmige Kante (111) umfasst, die in die Wand des Lochs (520) eingreift, wenn eine proximale Kraft auf den Anker (100) ausgeübt wird.

4. Ankersystem gemäß Anspruch 1, wobei die eine oder mehreren Anti-Backout-Vorrichtungen einen Widerhaken umfassen.

5. Ankersystem gemäß einem der Ansprüche 1 bis 4, wobei der Ankerkörper durch einen oder mehrere Bereiche mit reduzierter Materialdicke (130) segmentiert ist, die konfiguriert sind, um abgebrochen werden zu können, um die Länge des Ankerkörpers (100) zu reduzieren.

6. Ankersystem gemäß einem der vorhergehenden Ansprüche, wobei der Anker (2100) nahe dem distalen Ende (2101) des Ankerkörpers eine Falle (2800) durch den Ankerkörper umfasst, durch die ein Teil eines Weichgewebetransplantats (510) hindurchgeführt werden kann, wobei die Falle (2800) einer umgekehrten Hindurchführung des Weichgewebetransplantats (510) aus der Falle (2800) widersteht.

7. Ankersystem gemäß einem der vorhergehenden Ansprüche, wobei der Ankerkörper eine oder mehrere Ziffern (2900) umfasst, die auf dem Ankerkörper markiert sind, um eine Angabe über die Eindringtiefe des Ankers (2100) in das Loch (520) bereitzustellen.

8. Ankersystem gemäß einem der Ansprüche 1 bis 7, wobei der Verbindungsmechanismus ein Haltenahtmaterial (330) umfasst, das in der Lage ist, entfernbar zwischen der Einführungshilfe (200) und dem Anker (100) gekoppelt zu werden.

9. Ankersystem gemäß einem der Ansprüche 1 bis 7, wobei das distale Ende (225) des Schafts (220) so bemessen ist, dass es sich bis zu einem entfernten distalen Ende des Ankers (100) erstreckt, wenn sich der Anker (100) in Position auf der Einführungshilfe (200) befindet, um in das Loch (520) eingeführt zu werden.

10. Ankersystem gemäß Anspruch 1, wobei der Verbindungsmechanismus eine Reibungspassung zwischen der Einführungshilfe (200) und dem Anker (100) umfasst.

11. Ankersystem gemäß einem der vorhergehenden Ansprüche, das ferner ein Werkzeug (400) zur Reduzierung der Ankerlänge beinhaltet, das konfiguriert ist, um über ein proximales Ende (103) des Ankers (100) zu passen und verwendet zu werden, um eine Kraft auf den Anker (100) auszuüben, um die Länge des Ankers (100) zu reduzieren.

12. Ankersystem gemäß einem der vorhergehenden Ansprüche, das ferner ein Nahtmaterial (300) beinhaltet, das konfiguriert ist, um mit dem Anker (100) gekoppelt zu werden und mit einem Weichgewebetransplantat (510) gekoppelt zu werden.

## Revendications

1. Un système d'ancrage (1) comprenant :
une ancre (100) comprenant :
un corps d'ancre avec une extrémité distale (101), une extrémité proximale (103), et une longueur entre l'extrémité distale et l'extrémité proximale, et
un ou plusieurs éléments anti-retour (110) le long de la longueur du corps d'ancre configurés pour résister à un déplacement proximal de l'ancre lorsque l'ancre a été insérée dans un trou (520) avec un diamètre égal ou inférieur à un diamètre maximal de l'ancre (100),
dans lequel le corps d'ancre est segmenté au niveau d'un ou de plusieurs emplacements (130) le long de sa longueur de telle sorte que la longueur de l'ancre (100) peut être réduite en retirant l'extrémité proximale (103) et une portion de la longueur du corps d'ancre ; et
un inséreur (200) comprenant :
une poignée (210), et
un arbre (220) couplé à la poignée (201), l'arbre (220) incluant un mécanisme de liaison configuré pour se coupler à l'ancre de façon à pouvoir se libérer,
dans lequel le mécanisme de liaison inclut une extrémité distale (225) de l'arbre (220) qui s'étend le long d'une portion de l'ancre (100) et se met en contact avec celle-ci afin de soutenir l'ancre (100) ;
dans lequel le corps d'ancre de l'ancre (100) a une canulation (125) ;
dans lequel l'extrémité distale (225) de l'arbre (220) est dimensionnée pour s'emboîter au sein de la canulation (125) dans l'ancre (100) afin de fournir un soutien à l'ancre (100) ; et
**caractérisé en ce que** le corps d'ancre est segmenté par un ou plusieurs couplages (1130) entre des composants à encliqueter (1150), les composants à encliqueter (1150) étant configurés pour être détachés au niveau des un ou plusieurs couplages (1130) afin de réduire la longueur du corps d'ancre.

2. Le système d'ancrage de la revendication 1 dans lequel les un ou plusieurs éléments anti-retour incluent une dent (110).

3. Le système d'ancrage de la revendication 2 dans lequel la dent (110) est élastique et est configurée pour être éloignée par flexion d'une paroi du trou (520) lorsque l'ancre (100) est insérée dans le trou (520), et dans lequel la dent flexible (110) inclut une arête angulaire (111) qui s'accroche sur la paroi du trou (520) si une force proximale est exercée sur l'ancre (100).

4. Le système d'ancrage de la revendication 1 dans lequel les un ou plusieurs éléments anti-retour incluent une barbe.

5. Le système d'ancrage de n'importe laquelle des revendications 1 à 4 dans lequel le corps d'ancre est segmenté par une ou plusieurs zones d'épaisseur de matériel réduite (130) qui sont configurées pour être rompues afin de réduire la longueur du corps d'ancre (100).

6. Le système d'ancrage de n'importe quelle revendication précédente dans lequel l'ancre (2100) inclut une trappe (2800) à travers le corps d'ancre près de l'extrémité distale (2101) du corps d'ancre à travers laquelle une portion d'un greffon de tissu mou (510) peut être passée, la trappe (2800) résistant à un passage inverse du greffon de tissu mou (510) à partir de la trappe (2800).

7. Le système d'ancrage de n'importe laquelle des revendications précédentes dans lequel le corps d'ancre inclut un ou plusieurs numéros (2900) inscrits sur le corps d'ancre afin de fournir une indication d'une profondeur de pénétration de l'ancre (2100) dans le trou (520).

8. Le système d'ancrage de n'importe laquelle des revendications 1 à 7 dans lequel le mécanisme de liaison inclut une suture de fixation (330) capable de se coupler de façon amovible entre l'inséreur (200) et l'ancre (100).

9. Le système d'ancrage de n'importe laquelle des revendications 1 à 7 dans lequel l'extrémité distale (225) de l'arbre (220) est dimensionnée pour s'étendre jusqu'à une extrémité distale distante de l'ancre (100) lorsque l'ancre (100) est en position sur l'inséreur (200) à insérer dans le trou (520).

10. Le système d'ancrage de la revendication 1 dans lequel le mécanisme de liaison inclut un emboîtement par frottement entre l'inséreur (200) et l'ancre (100).

11. Le système d'ancrage de n'importe laquelle des revendications précédentes, comprenant en outre un outil de réduction de longueur d'ancre (400) configuré pour s'emboîter par-dessus une extrémité proximale (103) de l'ancre (100) et être utilisé pour exercer une force sur l'ancre (100) afin de réduire la longueur de l'ancre (100).

12. Le système d'ancrage de n'importe laquelle des revendications précédentes, comprenant en outre une suture (300) configurée pour être couplée à l'ancre (100) et pour être couplée à un greffon de tissu mou (510).
